# EUROPEAN PATENT SPECIFICATION

(11) **EP 1 682 047 B1**
(45) Date of publication and mention of the grant of the patent: **18.08.2010**
(21) Application number: 04769710.7
(22) Date of filing: 21.10.2004
(51) Int. Cl.: A61F 2/24

(54) **A prosthetic valve apparatus, in particular for cardiac application**
Klappenprothese, insbesondere für kardiale Anwendungen
Valve cardiaque

(30) Priority: 23.10.2003 IT BO20030631
(43) Date of publication of application: 26.07.2006
(73) Proprietor: Parravicini, Roberto Erminio, 41100 Modena (IT); Verona, Alessandro, 20124 Milano (IT)
(72) Inventor: PARRAVICINI, Roberto, Erminio, I-41100 Modena (IT); VERONA, Alessandro, I-20124 Milano (IT); CENTOLA, Marcos, 15025-010 San Paolo (BR)
(74) Representative: Jorio, Paolo
(86) International application number: PCT/IB2004/003480
(87) International publication number: WO 2005/039452

(56) References cited:
- WO-A-2004/058106
- CH-A5- 608 368
- US-A- 5 123 918
- US-A- 5 571 175
- US-B1- 6 652 540

## Description

### TECHNICAL FIELD

The present invention relates to the field of medical prosthetic apparatuses in general, and in particular to a prosthetic valve apparatus able to be implanted in a body district of the organism of humans or of mammals in general.

### BACKGROUND ART

The scope of the invention falls within the field of medicine, and in particular that of heart surgery, and it is aimed at the treatment of congenital or acquired valve pathologies for which it is indicated to replace with prosthesis one or more natural heart valves irreversibly compromised by pathological processes with different etiology.

The replacement of one or more heart valves with prostheses has long been a commonly accepted procedure. The surgery procedure normally entails the use of extra-body circulation, able to allow access to the cardiac cavities with the heart stopped and bloodless.

However, in spite of the advances made so far within the field of heart surgery, it can be stated that this type of operation is not free of possible complications able to induce morbidity or even mortality of the operated patients.

According to the general classification, currently two main categories of prosthetic substitutes of cardiac valves are clinically available:
(1) mechanical valve prostheses; and
(2) biological valve prostheses.

The specialist scientific literature describes in detail the types of prostheses, the indications pertaining to their selection, the surgical technique for their implant, possible complications and results.

However, it seems useful, to understand the subject and the innovative aspects included in the present invention, to provide a short foreword on the state of the art.

In the mechanical valve prostheses of the first type, the blood flow that traverses them is controlled by one or more shutters constitute by metallic rigid bodies assembled in such a way as to be able to oscillate on a respective rigid metallic annular support.

Instead, in the second case, i.e. in the case of biological valve prostheses (the so-called "bioprostheses"), the blood flow is controlled by strips constituted by biological tissue assembled on a rigid support.

In bioprostheses, the tissue used is generally of animal origin (bovine pericardium or native strips of porcine heart valve) preventively treated chemically to make it immunologically inert and structurally stable.

Among the biological tissues used for the construction of heart valves, a new biomaterial derived by corneal stroma of tuna has recently and advantageously been introduced with the aim of reducing or eliminating the possibility of structural failure of the bioprostheses over time.

In all cases (mechanical and biological apparatuses) as a structural component of the valve is included a component (the so-called "suture ring") assembled at the outer perimeter of the base of the prosthesis, able to house it and fasten it by means of suture stitches in orthotopic valve position instead of the previously excised native valve.

It is readily apparent that the presence of the suture ring at the outer perimeter of the base of the valve prosthesis determines a significant reduction in the effective area of the valve orifice.

Moreover, it is evident that the support of the biological strips (the so-called "valve stent", or simply "stent") also significantly limits the haemodynamic disengagement of the valve, causing a reduction in the useful area of opening of the prosthesis.

To make biological valve apparatuses anatomically more similar to natural heart valves and to improve their haemodynamic disengagement, numerous structural improvements have been introduced over time, giving rise to the sub-category of biological valve prostheses without support (so-called "stentless valves") and with suture ring positioned at the level of the base of the valve and not at the level of the outer perimeter of the prosthesis.

These solutions have enabled to obtain biological heart valve prostheses with a greater effective area of the valve orifice than traditional biological valves provided with stents. In spite of the evident haemodynamic benefits provided by the use of stentless biological valves, it is more difficult to implant this type of prosthesis than traditional biological valve prosthesis provided with stent and suture ring positioned at the outer perimeter of the stent.

This drawback is particularly reflected in the need for prolonged operating times with the heart stopped and in extra-body circulation and in the possibility of an incorrect positioning of the prosthesis at important structures adjacent to the implant site.

An additional drawback of biological prostheses in general, and of stentless ones in particular, is the impossibility of *in situ* orientation (i.e. after the implant), due to the absence of any system that allows the rotation of the valve prosthesis on the suture ring. This drawback is reflected in the absolute need to determine the correct orientation of the prosthetic apparatus during the operation, before executing the suture of the valve ring at the anatomic implant site.

In regard to the durability of the valve prostheses over time, it can be stated that mechanical valve prostheses have a duration that is practically unlimited over time due to the materials of construction, for example metal alloys which may be coated with pyrolithic carbon.

However, at the same time, mechanical valve prostheses have the disadvantage that the operated patient has to assume anti-coagulating and anti-aggregating thrombocytic drugs throughout his/her life, in order to prevent very sever, and even lethal, episodes of thromboembolism due to the contact of the blood with the metallic prosthetic material.

While biological valve prostheses do not have require the administration of anti-coagulating drugs, nonetheless have limited duration over time (from a few years in young patients, to a maximum of ten-fifteen years in older ones) due to the formation of calcium deposits within the valve strips, able to cause their structural failure.

The progressive deterioration of biological prostheses over time imposes a new surgical operation to replace the prosthesis with another valve prosthesis, entailing considerable operative risk in terms of morbidity and mortality of the operated patients.

In fact, in heart surgery it is not unusual to perform multiple interventions on the same patient after a time interval to replace deteriorated biological prostheses.

Therefore, the problems normally encountered when using traditional biological valve prostheses can be summarised as follows:
- limited duration over time, with the consequent need to replace the prosthesis;
- effective area of the valve orifice (useful area for the passage of the blood flow) limited by the presence of a peripheral suture ring external to the prosthesis;
- impossibility of orienting the valve prosthesis by rotation once it is sutured at the corresponding anatomical structures; and
- particularly challenging implant technique, with the need for a specific apprenticeship by the operator in the case of stentless biological valves.

The object of the present invention, therefore, is to overcome the important problems associated to currently existing and clinically available heart valve prostheses.

Moreover, the structural modifications introduced by the invention, in accordance with the main aspect thereof, make the surgical procedure for the implant simpler, more precise and safer.

### DISCLOSURE OF INVENTION

The invention relates to a heart valve prosthesis, in the current biological embodiment, but the construction of a mechanical valve prosthesis in accordance with the characteristics claimed in the claims is not precluded conceptually.

Therefore, a specific characteristic of the invention is to provide a modular valve prosthesis apparatus, in particular for cardiac applications, whose structural components are assembled together, as disclosed in Claim 1, in reversible fashion, by magnetic attraction with evident derived advantages.

"For example in WO 2004/058106 (ARBOR SURGICAL TECHNOLOGIES, INC.) it is described a valve prosthesis apparatus, in particular for cardiac applications, which comprises a valve prosthesis applied on a suture ring. Moreover, the valve prosthesis comprises, in its turn, a stent and an annular portion, and the valve prosthesis is fastened to the suture ring by magnetic means. However, the coupling of valve prosthesis on the suture ring in neither easy and nor precise because of the funnel-shaped coupling surfaces of the valve prosthesis and the suture ring.

Thus, the present invention is aimed to overcome the drawbacks presented by such piece of prior art."

By way of non limiting example, a possible embodiment of a biological valve prosthesis shall be illustrated below, although the concept can be extended to any type of valve prosthesis.

### BRIEF DESCRIPTION OF THE DRAWINGS

The present invention shall be described in detail with reference to the accompanying drawings, which illustrate and embodiment provided surely by way of non limiting example, in which:
- Figure 1 shows a three-dimensional global view of a valve prosthesis apparatus, in particular for cardiac applications, of the present invention, in this global view, the apparatus is shown in its "closed" configuration;
- Figure 2 shows a suture ring which is a part of the apparatus of Figure 1; and
- Figure 3 shows a three-dimensional global view of a stent, which is also a part of the apparatus of Figure 1.

### BEST MODE FOR CARRYING OUT THE INVENTION

For a better understanding of the terminology used in the detailed description of the present invention, a short explanation shall be provided below.
- Biological valve prosthesis: Substitute of a heart valve made, partially or totally, of a material of biological origin (generally, of animal origin). The most important structural components of a biological valve prosthesis are the "valve strips" made of biological material (bovine pericardium or porcine valve strips), and a "support structure" (the so-called "stent") made of plastic material; said "support structure" (stent) is not present in so-called stentless valves (without support).
- Suture ring: it is a ring made of synthetic material.

All these components ("valve strips", "stents", "suture ring") in traditional and known heart valve prosthesis apparatuses are assembled during their production, in such a way as to constitute a single body and non case can they be separated from each other in the subsequent steps without irreversible damaging the apparatus making it unusable for implant purposes.

As shown in the accompanying figures, a valve prosthesis apparatus 10 comprises a valve prosthesis 20 and a suture ring 30.

In turn, the valve prosthesis 20 comprises firstly a support structure (stent) 21, derived from stent that is a commonly used in the construction of biological prosthesis. The stent 21 is made of a rigid or preferably slightly flexible plastic material. Moreover, the stent 21 can be made of any other biocompatible material (steel, titanium, etc.).

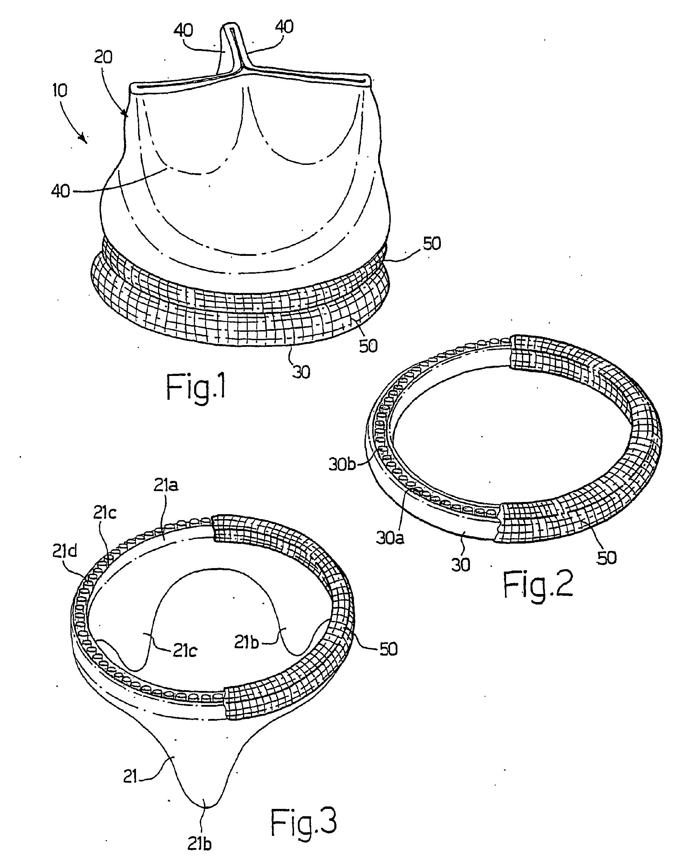

As shown in particular in Figure 3, the stent 21 comprises an annular portion 21a and three projecting portions 21b, advantageously positioned 120° from each other. Said projecting portions 21b are obtained integral with the annular portion 21a. At the base wall of the annular portion 21a, a continuous groove 21c is mechanical obtained; said groove 21c is able to house a plurality of permanent magnets 21d positioned mutually parallel.

In an embodiment not shown herein, instead of the plurality of permanent magnets 21d, it is possible to provide a single permanent magnet with annular shape or another geometry.

The permanent magnets 21d used in the embodiment described herein belong to the category of magnets provided with high attraction force (neodymium-iron-boron) and are coated with a material (epoxy resins or others) that is able to prevent their oxidation over time. The polarity of the magnets is oriented uniformly along the whole base of the stent 21 in such a way as to exert a homogeneous attraction of a corresponding structure positioned on the suture ring 30 which shall be described below.

The geometric shape of the base of the stent 21 is a circumference developed along a planar axis. In other embodiments, not shown herein, the geometry and the axis of the annular portion 21a of the stent 21 housing the magnets 21d can be modified.

The suture ring 30, in the current embodiment, exactly matches in its external and internal diameter the annular portion 21a of the stent 21. It is obtained by cutting the annular portion 21a of the stent 21 itself along a transverse axis. In this case, too, as shown in particular in Figure 2, a groove 30a is obtained mechanically; the groove 30a has the exact dimensions of the groove 21c obtained in the annular portion 21a of the stent 21, in order to house a similar plurality of magnets 30b arranged parallel to each other and with polarity oriented in such a way as to attract magnetically the magnets 21d positioned in the annular portion 21a of the stent 21.

Moreover, the suture ring 30 is coated by a synthetic tissue 50 for suturing the suture ring 30 to intracardial anatomic structures (valve annulus) (not shown).

The valve prosthesis 20 shown in Figure 1 has three valve strips 40 which can be made of any material of biological or synthetic origin.

Said valve strips 40 are assembled on the stent 21 with different procedures, normally by means of manually suture when they are fabricated. The valve strips 40 can also be housed within the stent 21 or be part of an integral structure "capped" outside the stent 21.

Advantageously, to obtain the valve strips 40 it is possible to use the corneal stroma of a fish, in particular of a tuna fish.

The surfaces of the annular portion 21a of the stent 21 and of the suture ring 30 are preferably coated with synthetic tissue 50 of such thickness as not to interfere or reduce magnetic attraction, and in such a way as not to create interruptions in the course of the subsequent magnetic coupling between the two modules of the apparatus 10 constituted by the valve prosthesis 20 and by the suture ring 30.

In particular, the synthetic tissue 50 positioned at the suture ring 30 extends for a short segment in order to be easily sutured to intracardiac structures (not shown).

Hereafter are described, by way of example, the standard manners of assembling the two main modules, i.e. the valve prosthesis 20 and the suture ring 30, during an aortic valve replacement. It is readily apparent for those skilled in the art that the same teachings can be applied to any tubular structure of the body and in particular to any heart valve (aortic, mitral, tricuspid, pulmonary).

The operation is normally performed through a median sternotomy, but other manners of accessing the heart are possible, such as lateral thoracotomy or other known ones. After cannulating the cardiac and vascular structures to allow the activation of extra-body circulation, the heart is arrested by infusion of a cardioplegic solution.

With the heart motionless and bloodless, the ascending aorta is transversely sectioned for a sufficient extension to provide an ample visualisation of the aortic valve and of the aortic annulus (peripheral circular anatomical portion where the three aortic valve strips are anchored in the transition point between left ventricle and origin of the aorta artery. After conducting the exeresis of the (pathological) valve strips at the insertion of the valve annulus, by means of a suitable instrument (sizer) the exact diameter of the valve annulus is measured for the selection of the prosthetic apparatus 10 having matching dimensions.

The subsequent phase, referred in particular to the invention, consists of suturing with surgical thread the magnetic suture ring 30 (module no. 1) of the apparatus 10 at the aortic valve annulus. In this phase, particular attention is paid in orienting the face of the suture ring 30 containing the magnets distally with respect to the heart in order to be subsequently able to attract the valve prosthesis 20 (module no. 2) magnetically.

It is readily apparent for those skilled in the art that the operating time for suturing the suture ring 30 at the aortic valve annulus is far simpler and more rapid than the traditional technique of suturing an entire valve apparatus to the annulus in accordance with the prior art. The valve prosthesis 20 (module no. 2), also magnetic, is then approached to the suture ring 30 (module no. 1) previously fastened to the annulus, causing a mutual magnetic attraction to be exerted due, as stated, to the presence of the magnets 21d, respectively 30b correctly oriented along the entire circumferences of the modules no. 1 and no. 2 having identical diameter.

The coupling by means of magnetic force is therefore immediate, secure, without interruptions between the two modules no. 1 and no. 2, coupled and without differences in diameter between them. The modular valve prosthesis apparatus thus assembled has the advantage of the ability to orient the module no. 2 *in situ* with respect to the module no. 1 (which is fixed). This is obtained by exerting a slight force inducing the sliding along the same axis of the module no. 2 on the module no. 1, without losing magnetic contact between said modules. The possibility of rotate the valve prosthesis 20 (module no. 2) *in situ* relative to the suture ring 30 (module no. 1) is particularly advantageous for a correct orientation in terms of aortic flow and of the optimal perfusion of the adjacent coronary ostia. Moreover, the apparatus 10 does not have a suture ring positioned at the periphery of the base of the stent, allowing the implant of a valve prosthesis with greater effective diameter than traditional apparatuses, with evident advantages of haemodynamic performance of the implant.

The prosthetic apparatus can be implanted via trans-catheter, i.e. without proceeding with a traditional heart surgery operation.

The prosthetic apparatus comprises, two main modules (module no. 1 and module no. 2), i.e. a valve prosthesis (module no. 2) and a suture ring (module no. 1).

In this case, the base of the valve prosthesis (module no. 2) is no longer a rigid structure in which the magnets are inserted, as in the embodiment already described above, but the magnets are sutured in the base of the biological tissue, thus allowing the possibility of temporarily reducing the diameter of the base of the valve prosthesis so it can be inserted in a catheter of smaller diameter than the original diameter of the prosthesis.

The suture ring (module no. 1) in this case is also not rigid, but the magnets are assembled within a synthetic material (polyester) which is also thin and easily deformed.

Therefore, in this case too it is possible simultaneously to reduce the diameter of the suture ring to allow its insertion in a catheter having considerably smaller diameter than the original diameter of the suture ring.

In practice, the two magnetic modules no. 1 and no. 2 are simultaneously reduced in size by compression and are inserted in series into an appropriate catheter. Under constant angiographic control, the catheter carrying the modules no. 1 and no. 2 is inserted into a peripheral artery or vein (e.g. femoral artery or femoral vein).

Once the heart is reached (in particular the site of the implant, aortic, pulmonary, mitral, tricuspid valve) the suture ring is released and expanded (with a balloon comprised in the catheter system) until it assumes its original diameter. Said suture ring is secured at the implant site (valve annulus).

The anchoring of the magnetic suture ring can be facilitated by the presence of hooks situated at the outer circumference of the module no. 1.

Immediately thereafter, the actual valve prosthesis is released. It is also expanded with a balloon comprised in the catheter system, returning to its original diameter, which exactly matches the diameter of the suture ring. By magnetic attraction, the two modules no. 1 and no. 2 are coupled in a manner that is exactly similar to the one described above for which, as stated, a traditional surgical technique is used.

To summarise, the advantages of the valve prosthesis apparatus of the present invention are the following:
(a) it can be implanted with an extremely simple, rapid and reliable technique;
(b) it can be replaced in case of need with a particularly simplified surgical procedure, in short operating times and with reduced risk for the patient;
(c) it can be rotated, in extremely simple and non traumatic fashion, *in situ* (after the implant) for a correct anatomical orientation;
(d) it assures better haemodynamic performance (corresponding to a greater effective area of the valve orifice) than existing prostheses;
(e) the capability of replacing the valve prosthesis (in particular, module no. 2, bearing the valve strips 40 which are normally subject to structural deterioration) without having to perform the exeresis of the suture ring (module no. 1) from the native valve annulus; to replace the module no. 2 leaving the module no. 1 *in situ,* it is sufficient to exert a force contrary to the magnetic attraction between two modules able to induce their separation (disassembly); it is evident that the operating phase necessary for the exeresis of the suture ring of a prosthesis from the heart and the suture of a new prosthesis entails more prolonged times, greater risks and likelihood of errors with respect to the mere replacement of the module no. 2 at the module no. 1 by simple and very rapid magnetic attraction; and
(f) the execution of the intervention to implant the valve prosthesis apparatus via trans-catheter allows considerable advantages for the operated patients; said implant technique does not require surgical access to the heart by means of sternotomy or thoracotomy, and it is not necessary to open the cardiac cavities, so it is not necessary to provide an implant which achieves an extra-body circulation; furthermore, an additional evident advantage of the trans-catheter implant is that it assures far more rapid recovery times than the traditional one.

## Claims

1. A valve prosthesis apparatus (10), in particular for cardiac applications, apparatus (10) comprising a valve prosthesis (20) applied on a suture ring (30);
wherein said valve prosthesis (20) comprises a stent (21) and an annular portion (21 a), and said valve prosthesis (20) being fastened to said suture ring (30) by magnetic means (21d, 30b);
apparatus (10) **characterised in that**
said magnetic means (21d, 30b) are contained in a respective continuous annular groove (21c, 30a) obtained, respectively, on the front surface of said annular portion (21a) and on the front surface of said suture ring (30);
said continuous annular groove (30a) on said suture ring (30) having the exact dimensions of said continuous annular groove (21 c) on said annular portion (21a), in order to house similar magnetic means (30b) presenting polarity oriented in such a way as to attract magnetically the magnetic means (21d) positioned in the groove (21c) of the annular portion (21a) of said valve prosthesis (20).

2. Apparatus (10) as claimed in Claim 1, **characterised in that** said magnetic means (21d, 30b) comprise a plurality of magnets (21d, 30b).

3. Apparatus (10) as claimed in Claim 1, **characterised in that** said magnetic means (21d, 30b) comprise magnets with annular shape.

4. Apparatus (10) as claimed in any of the previous claims, **characterised in that** said valve prosthesis (20) further comprises valve strips (40).

5. Apparatus (10) as claimed in claim 4, **characterised in that** said valve strips (40) are made of a biomaterial.

6. Apparatus (10) as claimed in claim 5, **characterised in that** said biomaterial is derived from corneal stroma, in particular from corneal stroma of tuna fish.

7. Apparatus (10) as claimed in any of the previous claims, **characterised in that** at least one portion of said valve prosthesis (20) and at least one portion of said suture ring (30) are coated by a synthetic tissue (50) able to facilitate the suture of said valve prosthesis (20) and of said suture ring (30).

8. Apparatus (10) as claimed in claim 1, **characterised in that** said valve prosthesis and said suture ring are made of deformable materials such as to allow their insertion into the body of a patient through a catheter, i.e. without having to proceed to a traditional heart surgery operation.

9. Apparatus (10) as claimed in claim 8, **characterised in that** the magnets are immersed in said deformable materials.

10. Apparatus (10) as claimed in either of the claims 8 or 9, **characterised in that** the suture ring is made of a synthetic material, in particular polyester.

11. Apparatus (10) as claimed in any of the claims 8, 9, 10, **characterised in that** the suture ring has a plurality of hooks able to facilitate its fastening in the implant site.

## Patentansprüche

1. Eine Klappenprothese (10), insbesondere für kardiale Anwendungen, wobei diese Prothese (10) eine Ventilprothese (20) enthält, die auf einem Nahtring (30) angeordnet ist;
wobei diese Klappenprothese (20) einen Stent (21) und einen ringförmigen Abschnitt (21 a) enthält und diese Ventilprothese (20) an diesem Nahtring (30) mittels magnetischer Einrichtung (21 d, 30b) befestigt ist;
diese Prothese (10) ist **dadurch gekennzeichnet, dass** die magnetischen Einrichtungen (21 d, 30b) in einer entsprechenden kontinuierlich verlaufenden ringförmigen Nut (21 c, 30a) enthalten und die auf der Frontoberfläche des ringförmigen Abschnittes (21 a) bzw. auf der Frontoberfläche des Nahtrings (30) vorhanden ist;
wobei die kontinuierlich ringförmige Nut (30a) auf dem Nahtring (30) die exakt gleichen Abmessungen der kontinuierlich verlaufenden ringförmigen Nut (21 c) auf diesem ringförmigen Abschnitt (21 a) aufweist, um ähnliche magnetische Einrichtung (30b) aufzunehmen, die Polaritäten derart aufweisen, dass die magnetischen Einrichtungen (21 d), die in der Nut (21 c) des ringförmigen Abschnittes (21 a) von dieser Klappenprothese (20) enthalten sind, angezogen werden.

2. Prothese (10) nach Anspruch 1, **dadurch gekennzeichnet, dass** die magnetischen Einrichtungen (21 d, 30b) eine Vielzahl von Magneten (21 d, 30b) enthalten.

3. Prothese (10) nach Anspruch 1, **dadurch gekennzeichneten, dass** die magnetischen Einrichtungen (21 d, 30b) Magneten mit ringförmiger Form aufweisen.

4. Prothese (10) nach irgendeinem der vorstehenden Ansprüche, **dadurch gekennzeichnet, dass** die Ventilprothese (20) des weiteren Ventilstreifen (40) aufweist.

5. Prothese (10) nach Anspruch 4, **dadurch gekennzeichnet, dass** die Ventilstreifen (40) aus einem Biomaterial hergestellt worden sind.

6. Prothese (10) nach Anspruch 5, **dadurch gekennzeichnet, dass** dieses Biomaterial von kornealem Stroma, insbesondere von kornealem Stroma eines Thunfisches erhalten worden ist.

7. Prothese (10) nach irgendeinem der vorstehenden Ansprüche, **dadurch gekennzeichnet, dass** zumindest ein Abschnitt dieser Ventilprothese (20) und zumindest ein Abschnitt dieses Nahtringes (30) mit einem synthetischen Gewebe (50) umhüllt sind, das in der Lage ist, diese Naht dieser Klappenprothese (20) und dieses Nahtringes (30) zu ermöglichen.

8. Prothese (10) nach Anspruch 1, **dadurch gekennzeichnet, dass** diese Ventilprothese (20) und dieser Nahtring (30) aus deformierbaren Materialien hergestellt worden sind, um **dadurch** ihre Einführung in den Körper eines Patienten durch einen Katheter zu ermöglichen, dass heißt, ohne eine traditionelle Herzoperation durchführen zu müssen.

9. Prothese (10) nach Anspruch 8, **dadurch gekennzeichnet, dass** die Magnete in diesem deformierbaren Material eingebettet sind.

10. Prothese (10) nach Anspruch 8 oder 9, **dadurch gekennzeichnet, dass** der Nahtring (30) aus einem synthetischen Material, insbesondere Polyester besteht.

11. Prothese (10) nach irgendeinem der Ansprüche 8, 9, 10, **dadurch gekennzeichnet, dass** der Nahtring (30) eine Vielzahl von Haken aufweist, die ihre Befestigung an der lmplantatseite unterstützend ermöglichen.

## Revendications

1. Dispositif de prothèse valvulaire (10), en particulier pour des applications cardiaques, le dispositif (10) comprenant une prothèse valvulaire (20) appliquée sur un anneau de suture (30) ;
dans lequel ladite prothèse valvulaire (20) comprend un stent (21) et une partie annulaire (21a), et ladite prothèse valvulaire (20) étant fixée sur ledit anneau de suture (30) par des moyens magnétiques (21d, 30b) ;
le dispositif (10) étant **caractérisé en ce que :**
lesdits moyens magnétiques (21d, 30b) sont contenus dans une rainure annulaire continue (21c, 30a) respective obtenue, respectivement, sur la surface avant de ladite partie annulaire (21a) et sur la surface avant dudit anneau de suture (30) ;
ladite rainure annulaire continue (30a) sur ledit anneau de suture (30) ayant les dimensions exactes de ladite rainure annulaire continue (21c) sur ladite partie annulaire (21a), afin de loger des moyens magnétiques similaires (30b) présentant une polarité orientée afin d'attirer magnétiquement les moyens magnétiques (21d) positionnés dans la rainure (21c) de la partie annulaire (21a) de ladite prothèse valvulaire (20).

2. Dispositif (10) selon la revendication 1, **caractérisé en ce que** lesdits moyens magnétiques (21d, 30b) comprennent une pluralité d'aimants (21d, 30b).

3. Dispositif (10) selon la revendication 1, **caractérisé en ce que** lesdits moyens magnétiques (21d, 30b) comprennent des aimants avec une forme annulaire.

4. Dispositif (10) selon l'une quelconque des revendications précédentes, **caractérisé en ce que** ladite prothèse valvulaire (20) comprend en outre des lames valvulaires (40).

5. Dispositif (10) selon la revendication 4, **caractérisé en ce que** lesdites lames valvulaires (40) sont réalisées avec un biomatériau.

6. Dispositif (10) selon la revendication 5, **caractérisé en ce que** ledit biomatériau est dérivé du stroma de la cornée, en particulier du stroma de la cornée du thon.

7. Dispositif (10) selon l'une quelconque des revendications précédentes, **caractérisé en ce qu'**au moins une partie de ladite prothèse valvulaire (20) et au moins une partie dudit anneau de suture (30) sont recouvertes par un tissu synthétique (50) pouvant faciliter la suture de ladite prothèse valvulaire (20) et dudit anneau de suture (30).

8. Dispositif (10) selon la revendication 1, **caractérisé en ce que** ladite prothèse valvulaire et ledit anneau de suture sont réalisés avec des matériaux déformables afin de permettre leur insertion dans le corps d'un patient par le biais d'un cathéter, c'est-à-dire sans avoir à procéder à une opération de chirurgie cardiaque classique.

9. Dispositif (10) selon la revendication 8, **caractérisé en ce que** les aimants sont immergés dans lesdits matériaux déformables.

10. Dispositif (10) selon l'une quelconque des revendications 8 ou 9, **caractérisé en ce que** l'anneau de suture est réalisé avec un matériau synthétique, en particulier du polyester.

11. Dispositif (10) selon l'une quelconque des revendications 8, 9, 10, **caractérisé en ce que** l'anneau de suture a une pluralité de crochets appropriés pour faciliter sa fixation dans le site d'implantation.
